(19) Europäisches Patentamt
European Patent Office
Office européen des brevets

(11) EP 0 746 294 B1

(12) **EUROPEAN PATENT SPECIFICATION**

(45) Date of publication and mention of the grant of the patent: **22.12.2004 Bulletin 2004/52**

(21) Application number: **94930744.1**

(22) Date of filing: **07.10.1994**

(51) Int Cl.[7]: **A61F 13/00**, A61L 15/00, A61K 47/18

(86) International application number: **PCT/US1994/011597**

(87) International publication number: **WO 1995/009590 (13.04.1995 Gazette 1995/16)**

(54) **ABSORPTION ENHANCERS FOR TOPICAL PHARMACEUTICAL FORMULATIONS**

ABSORPTIONSFÖRDERER FÜR TOPISCHE PHARMAZEUTISCHE FORMULIERUNGEN

COMPOSES POUR AMELIORER L'ABSORPTION TOPIQUE DE FORMULATIONS PHARMACEUTIQUES

(84) Designated Contracting States:
**AT BE CH DE DK ES FR GB GR IE IT LI LU MC NL PT SE**

(30) Priority: **07.10.1993 US 133454**

(43) Date of publication of application: **11.12.1996 Bulletin 1996/50**

(73) Proprietor: **ODONTEX, INC.**
**Lawrence, KS 66049 (US)**

(72) Inventors:
- **BÜYÜKTIMKIN, Servet Apartment 13**
  **Lawrence, KS 66044 (US)**
- **BÜYÜKTIMKIN, Nadir 2partment 13**
  **Lawrence, KS 66044 (US)**
- **RYTTING, Joseph, Howard**
  **Lawrence, KS 66047 (US)**

(74) Representative: **Vossius, Volker, Dr. et al**
**Dr. Volker Vossius,**
**Patentanwaltskanzlei - Rechtsanwaltskanzlei,**
**Geibelstrasse 6**
**81679 München (DE)**

(56) References cited:

| | |
|---|---|
| **WO-A-92/16236** | **US-A- 4 731 241** |
| **US-A- 4 732 892** | **US-A- 4 808 414** |
| **US-A- 4 980 378** | **US-A- 5 082 866** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Cross-Reference to Related Application

This application is a continuation-in-part of co-pending U.S. patent application Serial No. 08/133,454, filed October 7, 1993.

### Technical field

The invention relates to the development of safe and effective agents which improve the rate of percutaneous and oral mucosal transport of physiologically active agents. More particularly, the present invention relates to an improved penetration enhancer for use in the delivery of a local or systemic physiologically active agent to a mammalian organism.

### Background of the invention

Dermal drug formulations may represent the oldest drug dosage form in human history. It is highly probable that even ancient people used resins and animal fats to treat damage to the skin resulting from injuries and burns. The use of such dermal formulations for local effect remained largely unchanged until the middle of this century. The concept of administering drugs through the skin to achieve a local or systemic effects was first seriously advocated in the early 1970's. Since that time extensive research has been undertaken in this field.

The transdermal route of drug administration offers a number of advantages over the more conventional routes of drug administration. For instance, a drug may be delivered to targeted tissues from adjacent skin areas. The transdermal route of drug administration also allows for a gradual, controlled release of. drug into the systemic circulation. Since many drugs are poorly absorbed or delivered through the traditional routes of administration, the transdermal route provides an effective method of achieving improved bioavailability for those drugs. The transdermal route of drug administration is also advantageous since the administration of dermally administered drugs may be easily stopped should an undesirable side effect occur during therapy.

In spite of the foregoing advantages, transdermal formulations are limited. They cannot be used with most polar drugs since they tend to penetrate the skin too slowly. This characteristic is particularly crucial since most drugs are of a polar character. In addition, many drugs elicit a reaction and/or irritation at the site of topical application.

Two methods are known for improving the rate of penetration of polar drugs across the skin. The first method is to make a better formulation of the drug to increase its thermodynamic activity. The thermodynamic activity of a drug in a dermal formulation is dependent on the concentration of the drug and the choice of vehicle. The second method involves the use of physical methods, e.g., iontophoresis, or chemical compounds, e.g., penetration enhancers, to increase the permeability of the barrier membrane. The latter method is generally more practical because of its convenience and effectiveness.

Thus, in the last two decades a wide variety of compounds have been evaluated as to their effectiveness in enhancing the rate of penetration of drugs through the skin. The classically recognized strong enhancers tend to be proton accepting solvents, e.g., dimethyl sulfoxide (DMSO) and dimethyl acetamide (DMA). Recently, 2-pyrrolidone, N,N-diethyl-m-toluamide (DEET), 1-dodecylazacycloheptane-2-one (Azone® a registered trademark of Nelson Research), N,N-dimethylformamide, N-methyl-2-pyrrolidone and calcium thioglycolate have been reported as effective enhancers.

In previous work of some of the present co-inventors as reported in U.S. Pat. No. 4,980,378, issued December 25, 1990 and in U.S. Pat. No. 5,082,866, issued January 21, 1992, a group of biodegradable absorption enhancers which are alkyl N,N-disubstituted amino acetates were reported. These compounds were shown to be highly effective biodegradable penetration enhancers for the percutaneous delivery of clonidine and indomethacin.

WO 92/16236 discloses a method and compositions for enhancing absorption of topically administered physiologically active agents through the skin and mucous membranes of humans and animals in a transdermal device or formulation for local or systemic use, comprising a therapeutically effective amount of a pharmaceutically active agent and a non-toxic, effective amount of an aminoalcohol derivative as a penetration enhancing agent.

US-4.731,241 discloses a percutaneous pharmaceutical preparation for external use containing N-ethoxycarbonyl-3-morpholinosydnonimin (molsidomine) and an absorption promoter selected from the group consisting of aliphatic monocarboxylic acids of 5 to 30 carbon atoms, aliphatic monoalcohols of 10 to 22 carbon atoms, aliphatic monoamides of 8 to 18 carbon atoms and aliphatic monoamines of 10 to 16 carbon atoms, which gives a high blood concentration of molsidomine over a long time period when applied onto the human skin.

The biodegradable absorption enhancers as reported in the above identified patents solve several of the problems that have been associated with many prior art dermal enhancers. Included among those problems is the fact

that they could not be used with most polar drugs because they tended to penetrate the skin too slowly. Moreover, many of the prior art dermal enhancers produced reactions and/or irritation at the site of application. Thus, these earlier patents, commonly assigned with this application, represented an improvement solving some problems.

We have now discovered that compounds which are N,N-disubstituted amino alkanol esters of certain aliphatic acids show unexpectedly good penetration enhancing properties. For example, a representative of this class of compounds is a n-alkanoic acid derivative of an amino alkanol, 1-(N,N-dimethylamino)-2-propanol dodecanoate, which can be prepared by reacting the corresponding amino alkanol with lauroyl chloride in the presence of triethylamine. These compounds are easy to prepare in high yields.

Accordingly, it is a primary objective of the present invention to provide additional biodegradable absorption enhancers that substantially increase the thermodynamic activity of pharmacologically active agents and enhance the permeability of barrier membranes, such as skin or mucous membranes.

Another objective of the present invention is to provide an improvement of transdermal drug delivery by providing additional novel compounds which enhance the absorption of active substances through the skin and mucous membranes such as the gingivae. These drugs have reduced toxicity and lower preparation cost than those compounds that have been normally used in the past.

The method of accomplishing these and other objectives of the invention will become apparent from the detailed description of the invention which follows hereinafter.

## SUMMARY OF THE INVENTION

A novel group of esters of long chain aliphatic acids with N,N-disubstituted amino alcohols have been designed and prepared. The long chain acid moiety may be a residue of either a long chain saturated carboxylic acid or an unsaturated carboxylic acid, such as oleic or linoleic acid. These compounds, whether saturated (I) or unsaturated (II) provide a new class of biodegradable (or "soft") penetration enhancers which, because of their structure, have less adverse or toxic effects but which are nevertheless excellent promoters of percutaneous and oral mucosal (especially gingival) absorption.

The present invention also provides a method for introducing physiologically active agents through body surfaces such as skin and mucous membranes and for compositions for use in that method. More specifically, the invention relates to a method for increasing the penetration of a physiologically active agent across the skin of a mammalian organism by topically applying a physiologically active agent in an amount sufficient to achieve the desired local or systemic effect along with the biodegradable (or "soft") penetration enhancing compound described above in an amount sufficient to effectively increase penetration of the physiologically active agent. The invention further provides a pharmaceutical composition comprising one or more of these compounds along with a pharmaceutically acceptable carrier therefor.

## BRIEF DESCRIPTION OF THE DRAWINGS

FIG. 1 is a graph of the penetration profiles (cumulative amount of drug detected in the receptor cell of a vertical diffusion cell apparatus versus time) for solutions containing clonidine in the presence of an enhancer of the present invention, 1-(N,N-dimethylamino)-2-propanol dodecanoate [DAIPD (■)], the prior art enhancer Azone® (□) and control solutions lacking enhancer (▲).

FIG. 2 is a graph of the penetration profiles for solutions containing hydrocortisone in the presence of DAIPD (■), Azone® (□) and a control (▲).

FIG. 3 is a graph of the penetration profiles for solutions containing indomethacin in the presence of DAIPD (■), Azone® (□) and a control (▲).

FIG. 4 is a graph of the penetration profiles for solutions containing clonidine in the presence of another enhancer of the present invention, 1-(N,N-dimethylamino)-2-propanol myristate (DAIPM, ■), Azone® (□) and a control (▲) ; means ± standard deviation (SD), n=3-5.

FIG. 5 is a graph of the penetration profiles for solutions containing hydrocortisone in the presence of DAIPM (■), Azone® (□) and a control (▲); means ± SD, n=3-5.

FIG. 6 is a graph of the penetration profiles for solutions containing indomethacin in the presence of DAIPM (■), Azone® (□) and a control (▲); means ± SD, n=3-5.

FIG. 7 is a graph of the penetration profiles for solutions containing prostaglandin $E_1$ in the presence of DAIPM (■), Azone® (□) and a control (▲); means ± SD, n=3-5.

FIG. 8 is a graph of the penetration profiles for 45% ethanolic solutions containing Prazosin after pretreatment with DAIPM (■), Azone® (□) and a control (▲).

Figure 9 shows the biodegradability of DAIPD in the presence of porcine esterase at 32° C at a pH of 7.0.
Figure 10 is a graph of the penetration profiles for solutions containing clonidine in the presence of an unsaturated ester of the invention, DAIPO (○), dodecyl N,N-dimethylamino acetate [DDAA (•)], oleic acid (□) and a control (▲).

## DETAILED DESCRIPTION OF THE INVENTION

As earlier mentioned, the compounds of the present invention are esters of a long chain acid, which may be saturated (formula I) or unsaturated (formula II), with an amino alcohol. Where the long chain moiety is saturated, the compounds have the formula:

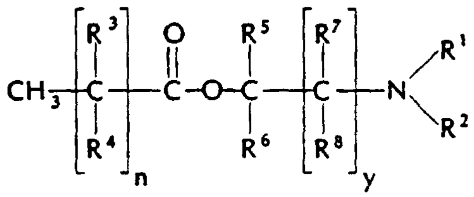

(I)

wherein n is an integer having a value in the range of 5 to 18; y is an integer having a value in the range of 0 to 5; and $R^1$, $R^2$, $R^3$, $R^4$, $R^5$, $R^6$, and $R^7$ can be alike or different from one another and are members of the group consisting of hydrogen, and $C_1$- to $C_8$- alkyl; and $R^8$ is a member of the group consisting of hydrogen, hydroxyl and $C_1$- to $C_8$- alkyl.

Examples of suitable compounds of formula (I) of this invention are listed in TABLE 1.

TABLE 1

| Formula (I) Penetration Enhancers | |
|---|---|
| (1) | $CH_3$-$(CH_2)_{10}$-CO-O-$CH_2$-$N(CH_3)_2$ |
| (2) | $CH_3$-$(CH_2)_{11}$-CO-O-CH$(CH)_3$-$N(CH_3)_2$ |
| (3) | $CH_3$- $(CH_2)_{10}$CO-O-$CH_2$-$CH_2$-$N(CH_3)_2$ |
| (4) | $CH_3$- $(CH_2)_{10}$-CO-O-$CH_2$-$CH_2$-$CH_2$-N $(CH_3)_2$ |
| (5) | $CH_3$ - $(CH_2)_{10}$-CO-O-CH $(CH_3)$ -$CH_2$-$N(CH_3)_2$ |
| (6) | $CH_3$- $(CH_2)_{10}$-CO-O-$CH_2$-$CH(CH_3)$-$N(CH_3)_2$ |
| (7) | $CH_3$- $(CH_2)$ $_{10}$-CO-O-$CH_2$-C $(CH_3)$ $_2$-N $(CH_3)$ $_2$ |
| (8) | $CH_3$-$(CH_2)_{10}$-CO-O-$CH_2$-CH(OH)-$CH_2$-$N(CH_3)_2$ |
| (9) | $CH_3$-$(CH_2)_{10}$-CO-O-$(CH_2)_4$-$N(CH_3)_2$ |
| (10) | $CH_3$- $(CH_2)_{10}$-CO-O- $(CH_2)_6$-$N(CH_3)_2$ |
| (11) | $CH_3$-$(CH_2)_8$-CO-O-$CH(CH_3)$-$CH_2$-$N(CH_3)_2$ |
| (12) | $CH_3$-$(CH_2)_{12}$-CO-O-$CH(CH_3)$-$CH_2$-$N(CH_3)_2$ |
| (13) | $CH_3$-$(CH_2)_3$-CH $(CH_3)$-$(CH_2)_2$-CO-O-$CH(CH_3)$-$CH_2$-$N(CH_3)_2$ |
| (14) | $CH_3$- $(CH_2)_4$-$CH(CH_3)$ -CO-O-$CH(CH_3)$ -$CH_2$-$N(CH_3)_2$ |
| (15) | $CH_3$-CH $(CH_3)$-$(CH_2)_2$-CO-O-$CH(CH_3)$-$CH_2$-N $(CH_3)_2$ |

Preferably $R^1$ and $R^2$ are selected from the group consisting of hydrogen, methyl and ethyl and more preferably $R^1$ and $R^2$ are methyl. Where the compound of formula (I) is a saturated long chain moiety, the preferred enhancer prepared is 1-(N,N-dimethylamino)-2-propanol dodecanoate [DAIPD, Table 1 (5)]. Another preferred formula (I) enhancer is 1-(N,N-dimethylamino)-2-propanol myristate [DAIPM, Table 1 (12)].

While the alkyl radicals may be straight or branched, i.e., methyl, ethyl, propyl, butyl, pentyl, hexyl, heptyl, and octyl, it is preferred that they be straight chain since these seem to provide the greatest enhancement.

When the compounds are prepared from unsaturated long fatty acid chains, they have the formula:

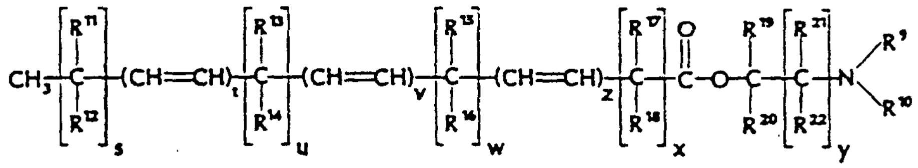 (II)

wherein t, v, and z each is an integer having a value in the range of 0 to 1 inclusive, such that (t + v + z) equals an integer of at least 1, s, u, w, and x each is an integer having a value in the range of 0 to 12 inclusive, such that (s + u + w + x) equals an integer in the range of 4 to 18 inclusive, y is an integer having a value in the range of 0 to 5 inclusive, and $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, and $R^{22}$ can be alike or different from one another and are members of the group consisting of hydrogen, and $C_1$- to $C_9$- alkyl.

For compounds of formula (II), preferably $R^9$ and $R^{10}$ are members of the group consisting of hydrogen, methyl and ethyl, more preferably $R^9$ and $R^{10}$ are methyl. A particularly preferred formula (II) compound is 1-(N,N-dimethylamino) -2-propanol oleate (DAIPO), i.e., a compound of formula (II) where s and x have the value of 7, t has the value of 1, u, v, w, and z are each zero, y is 1, $R^{11}$, $R^{12}$, $R^{17}$, $R^{18}$, $R^{19}$, $R^{21}$ and $R^{22}$ are each hydrogen, and $R^9$, $R^{10}$, and $R^{20}$ are methyl.

The compounds of formula (I) or formula (II) are readily prepared by a one-step synthesis by reacting the corresponding aminoalkanol with alkanoyl or alkenoyl halide, preferably chloride, in the presence of triethylamine, typically in a suitable solvent such as chloroform. The reaction may be conducted in the presence of a solvent or not, since the solvent is optional.

The amount of the penetration enhancer of formula (I) which may be used in the present invention is an effective, non-toxic amount for enhancing absorption either through the skin or mucous membranes. Generally, this amount ranges from about 0.4 weight percent to about 95 weight percent of the topical composition. Preferably, about 0.5 to about 40 weight percent of the penetration enhancer, based on the total weight of the composition, is used.

The compounds described herein are useful in improving percutaneous or mucous membrane absorption of physiologically active agents. The term "percutaneous" as used herein means transdermal or transcutaneous; it denotes passage of substances through unbroken skin. While the term "mucous membrane absorption" refers generally to any of the mucous membranes in the body, absorption through the mucous membranes of the oral cavity is of particular interest. Thus, buccal, sublingual, gingival and palatal absorption are specifically contemplated by the present invention. In a preferred embodiment, the instant penetration enhancers are used to improve absorption through those oral tissues which most resemble the skin in their cellular structure, i.e., the gingivae and palate. The term "physiologically active agent" is used herein to refer to a broad class of useful chemical and therapeutic agents including physiologically active steroids, antibiotics, antifungal agents, antibacterial agents, antineoplastic agents, analgesics and analgesic combinations, anorexics, anthelmintics, antiarthritics, antiasthia agents, anticonvulsants, antidepressants, antidiabetic agents, antidiarrheals, antihistamines, anti-inflammatory agents, antimigraine preparations, antimotion sickness preparations, antinauseants, antiparkinsonism drugs, antipruritics, antipsychotics, antipyretics, antispasmodics, including gastrointestinal and urinary; anticholinergics, sympathomimetics, xanthine derivatives, cardiovascular preparations including calcium channel blockers, betablockers, antiarrhythmics, antihypertensives diuretics, vasodilators including general, coronary, peripheral and cerebral; central nervous system stimulants, cough and cold preparations, decongestants, hormones, hypnotics, immunosuppressives, muscle relaxants, parasympatholytics, parasympathomimetics, psychostimulants, sedatives, tranquilizers, allergens, antihistaminic agents, anti-inflammatory agents, physiologically active peptides and proteins, ultraviolet screening agents, perfumes, insect repellents, hair dyes, and the like. The term "physiologically active" in describing the agents contemplated herein is used in a broad sense to comprehend not only agents having a direct pharmacological effect on the host but also those having an indirect or observable effect which is useful in the medical arts, e.g., the coloring or opacifying of tissue for diagnostic purposes, the screening of ultraviolet radiation from the tissues and the like.

For instance, typical fungistatic and fungicidal agents include thiabendazole, chloroxine, amphotericin, candicidin, fungimycin, nystatin, chlordantoin, clotrimazole, ethonam nitrate, miconazole nitrate, pyrrolnitrin, salicylic acid, fezatione, ticlatone, tolnaftate, triacetin, zinc, pyrithione and sodium pyrithione.

Steroids include cortisone, cortodoxone, fluoracetonide, fludrocortisone, difluorsone diacetate, flurandrenolone acetonide, medrysone, amcinafel, amcinafide, betamethasone and its esters, chloroprednisone, clorcortelone, descinolone, desonide, dexamethasone, dichlorisone, difluprednate, flucloronide, flumethasone; flunisolide, fluocinonide, flucortolone, fluoromethalone, fluperolone, fluprednisolone, meprednisone, methylmeprednisone, paramethasone, prednisolone and predisone.

Antibacterial agents include sulfonamides, penicillins,cephalosporins,penicillinase,erythromycins, linomycins, vancomycins, tetracyclines, chloramphenicols, streptomycins, and the like. Specific examples of antibacterials include erythromycin, erythromycin ethyl carbonate, erythromycin estolate, erythromycin glucepate, erythromycin ethylsuccinate, erythromycin lactobionate, lincomycin, clindamycin, tetracycline, chlortetracycline, demeclocycline, doxycycline, methacycline, oxytetracycline, minocycline, and the like.

Peptides and proteins include, in particular, small to medium-sized peptides, e.g., insulin, vasopressin, oxytocin and human growth hormone.

Other agents include iododeoxyuridine, podophyllin, theophylline, isoproterenol, triamcinolone acetonide, hydrocortisone, indomethacin, phenylbutazone paraaminobenzoic acid, aminopropionitrile and penicillamine.

The foregoing list is by no means intended to be exhaustive, and any physiologically active agent may be applied by the method of the present invention.

An important advantage of the present invention is that absorption of polar bioactive agents as well as non-polar drugs is also improved. The polar bioactive agents encompass a variety of therapeutic agents such as the xanthines, triamterene and theophylline, the antitumor agents, 5-fluorouridinedeoxyriboside, 6-mercaptopurinedeoxyriboside, vidarabine, the narcotic analgesics, hydromorphone, cyclazine, pentazocine, bupomorphine, the compounds containing organic anions, heparin, prostaglandins and prostaglandin-like compounds, cromolyn sodium, carbenoxolone, the polyhydroxylic compounds, dopamine, dobutamine, 1-dopa, a-methyldopa, the polypeptides, angiotensin antagonists, bradykinin, insulin, adrenocorticotrophic hormone (ACTH), enkephalins, endorphins, somatostatin, secretin and miscellaneous compounds such as tetracyclines, bromocriptine, lidocaine, cimetidine or any related compounds. The quantity of these polar bioactive agents necessary for preparing the drug form could vary over a wide range, but would normally be regulated by that quantity necessary to comprise the therapeutically effective dosage form.

Agents normally applied as eye drops, ear drops or nose drops or into the membranes of the oral cavity are also more effective when applied along with the penetration enhancers of the present invention.

As indicated earlier, agents used in diagnosis may be used more effectively when applied dissolved in one of the vehicles of this invention. Patch tests to diagnose allergies may be effected promptly without scratching the skin or covering the area subjected to an allergen when the allergens are applied along with the enhancers of this invention.

This invention is also useful in topical application of cosmetic or aesthetic agents. For example, compounds such as melanocyte-stimulating hormone (MSH) or dihydroxyacetone and the like are more effectively applied to skin to simulate a suntan when they are applied along with the enhancers of this invention. The agent is carried into the skin more quickly and in greater quantity when applied in accordance with this invention. Hair dyes also penetrate more completely and effectively when dissolved in one of the vehicles of this invention.

While the foregoing discussion describes the simultaneous administration of the physiologically active agent along with the penetration enhancer, the penetration enhancer may be applied before or after the application of the physiologically active agent, if desired.

The physiologically active agents intended for use in the practice of the present invention are intended for either their systemic or their local effect.

Dosage forms for topical application to the skin or mucous membranes of humans and animals include creams, lotions, gels, ointments, suppositories, sprays, for example, nasal sprays, aerosols, buccal and sublingual tablets, gingival and buccal patches or any one of a variety of transdermal devices for use in the continuous administration of systemically active drugs by absorption through the skin, oral mucosa or other membranes. See for example, one or more of U.S. Pat. Nos. 3,598,122, 3,598,123, 3,731,683, 3,742,951, 3,814,097, 3,921,636, 3,971,376, 3,993,072, 3,993,073, 3,996,934, 4,031,894, 4,060,084, 4,069,307, 4,201,211, 4,230,105, 4,292,299 4,292,303 and 4,077,407. The foregoing patents also disclose a variety of specific systematically active agents which may also be useful in transdermal delivery, which disclosures are hereby incorporated herein by reference.

The usual pharmaceutical compounding agents, diluents or carriers may be included in these compositions as desirable for the particular route of administration and dosage form. The amount and type of diluent or carrier used should, of course, be consistent with the compatibility of the agent with the compound of this invention. For instance, a cosolvent or other standard adjuvant, such as a surfactant, may be necessary to maintain the agent in solution or suspension at the desired concentration.

For nasal sprays and other mucous membrane applications isotonic saline may be preferable as a diluent. The instant enhancer may be present in these forms in various concentrations, for example, from about 2% to about 75% by weight or higher.

Lotions and gels, ointments or creams, may contain the usual ingredients to provide a base, as for example cetyl alcohol, or an emulsifier such as lauryl sulfate and water. Another base may be formulated by combining equal weight amounts of stearic acid, cetyl alcohol, triethanolamine and glycerol monostearate with water. Still other bases may utilize polyethylene glycols of different viscosities, depending upon the desired consistency.

A suppository form may be made from a high viscosity polyethylene glycol 4000, water and the penetration enhancer.

Typical inert carriers which may be included in the foregoing dosage forms include conventional formulating

materials such as, for example, water, acetone, isopropyl alcohol, halogenated hydrocarbons (freons), ethyl alcohol, polyvinyl pyrrolidone, propylene glycol, fragrances, gel-producing materials such as "Carbopol", mineral oil, stearyl alcohol, stearic acid, spermaceti, sorbitan monooleate, "Polysorbates", "Tweens", sorbitol, methylcellulose, and the like. The compositions of the present invention are formulated with any suitable nontoxic pharmaceutically acceptable inert carrier material. Such carrier materials are well known to those skilled in the art of pharmaceutical formulations. For those not skilled in the art, reference is made to the text entitled Remington's Pharmaceutical Sciences, 18th Edition, 1990, ed. Alfonso R. Gennaro, Mack Publishing Company, Easton, Pa.

Any type of transdermal drug delivery system is suitable for the practice of the present invention, for instance, a transdermal patch, a buccal tablet, and the like. A variety of transdermal drug delivery systems are disclosed and described in U.S. Pat. No. 4,624,665, herein incorporated by reference.

The amount of the composition, and thus of the physiologically active agent therein to be administered will be an effective amount for the desired result expected therefrom. This, of course, will be ascertained by the ordinary skill of the practitioner. Due to the enhanced activity which is achieved, the dosage of the physiologically active agent may often be decreased from that generally applicable. In accordance with the usual prudent formulating practices, a dosage near the lower end of the useful range of the particular agent may be employed initially and the dosage increased as indicated from the observed response, as is the routine procedure of the physician.

The concentration of the physiologically active agent in the various dosage forms is, of course, commensurate with that normally utilized for the particular agent in conventional formulations for effective results for the intended route. Both the amount of physiologically active agent and the amount of penetration enhancer will be influenced by the type of effect desired. To a certain degree, if a more localized effect is required, as for example, in treating a superficial infection with an antibacterial agent, lower amounts of physiologically active agents and lower concentrations of enhancer may be called for. Where deeper penetration is desired, as in the case of local anesthesia, a higher concentration of enhancer may be desirable to promote adequate penetration. Where general systemic concentration of an agent is desired for a topical preparation, generally higher concentrations of enhancer are desirable and the amount of agent as, for example, a steroid, may be included in the composition sufficient to provide the blood level desired.

## DESCRIPTION OF THE PREFERRED EMBODIMENTS

In order to further illustrate the present invention and the advantages thereof, the following specific examples are given, it being understood that the same are intended only as illustrative and in nowise limitative.

### EXAMPLE 1

### Penetration Promoting Ability of 1-(N,N-dimethylamino)-2-propanol dodecanoate (DAIPD)

The penetration promoting activity of DAIPD was compared to that of Azone®, taken as a typical enhancer, using shed snake skin as a model membrane. The penetration profiles of clonidine, hydrocortisone and indomethacin in pH 7.0 buffer and in the presence of DAIPD and Azone® at 32°C are shown in FIG. 1 - FIG. 3 and TABLE 2 - TABLE 4. The permeants were selected to provide examples of basic drugs (clonidine), neutral drugs (hydrocortisone) and acidic drugs (indomethacin).

To 20.6 g (0.2 M) of 1-(N,N-dimethylamino)-2-propanol in 250 ml $CHCl_3$ in the presence of triethylamine (30 ml), 43.2 g (0.2 M) of lauroyl chloride was added incrementally and stirred for 24 hours at room temperature. After filtration of the residue, the reaction mixture was washed three times with water (250 ml each washing) and the organic phase was dried over anhydrous magnesium sulfate. The solvent was evaporated in vacuo, the oily residue was dissolved in ethyl acetate, and purified by column chromatography using the same solvent. The reaction course was checked by thin layer chromatography (TLC). Ethyl acetate was used as the solvent for TLC analysis. The visualization was accomplished using iodine vapors. The yield was 95%. The TLC Rf values were 0.21 (ethyl acetate) and 0.74 (chloroform/methanol (1:1)). Infrared (IR), nuclear magnetic resonance (NMR) and mass (electron ionization) spectra (MS) were determined and included. IR ($CHCl_3$) : $\gamma$2920, 2840 (C-H), 1730 (C=O), 1040 (C-O-C) $cm^{-1}$; 1H-NMR ($CDCl_3$) $\delta$ 0.88 (3H, t, $CH_3$) , 1.21-1.23 (3H, d, $CH_3$-CH), 1.26 (broad s, 18H, $(CH_2)_9$) , 2.25 (6H, s, $N(CH_3)_2$), 2.29 (2H, t, $CH_2$-N) , 2.46-2.52 (2H, m, $CH_2$-CO), 5.02-5.08 (1H, m, CH-$CH_3$) ppm; MS (EI) : m/z (RA %) 285 (10), 158 (45), 145(70), 102 (28), 58(100); $C_{17}H_{35}NO_2$ (285.47) requires 285.

The lipophilicity expressed as Rm values was determined according to the method of Seydel and Schaper (Seydel, J.K., and Schaper, K.J., Chemische Struktur und biologishe Aktivitaet von Wirkstoffen, Verlag Chemie, Weinheim, New York, 1979, pp 257-259). The plates were impregnated with 5% paraffin solution in diethylether. A mixture of acetone and phosphate buffer (0.01 M, pH 7.0) (4:1) was used as the developing system. The Rm value was calculated by the use of the following equation:

$$Rm = \log[(1/Rf)-1]$$

The apparent pKa values were determined by titration of 0.25 mM of enhancer using aqueous 0.1 N HCl as titrant in 50 ml acetonitrile/water mixture (1:1). They were calculated according to Albert and Serjeant (Albert, A. and Serjeant, E.P., The Determination of Ionization Constants, Chapman and Hall, London, 1971 pp 39-40) .

Shed snake skins were prepared as previously reported (Büyüktimkin, S., Büyüktimkin, N., and Rytting, J.H., Synthesis and enhancing effect of dodecyl 2-(N,N-dimethylamino) propionate on the transepidermal delivery of indomethacin, clonidine, and hydrocortisone, Pharm. Res., 10: 1632-1637, 1993) and as described in U.S. Patents No. 4,980,378 and No. 5,082,866, the disclosures of which are incorporated herein by reference. Shed snake skins were stored at -20°C. Before the experiment they were allowed to come to room temperature at least 12 hours before use. To reduce the variability of results, pieces cut from the same whole snake skin were used for each set of experiments

Indomethacin, clonidine, and hydrocortisone were assayed by HPLC procedures as described earlier; see Büyüktimkin et al., (1993), above.

Pieces of shed snake skin (approximately 3 x 3 cm) were pretreated with 15 µl of enhancer (divided into three 5 µl portions) 2 hr before the experiment. After mounting the skin on top of a Franz receptor cell filled with pH 7.0 (0.1 M) phosphate buffer, the donor cell was clamped on the top of the receptor cell. An aliquot of 0.5 ml of a suspension of hydrocortisone or indomethacin, prepared by suspending 50 mg or 25 mg of the drugs respectively, in 25 ml of the same buffer and stirring for 24 hr at 32°C, or a solution of clonidine (2%) in the same buffer were added to the donor cell. The solution in the receptor compartment was stirred by a magnetic stirrer. At appropriate time intervals samples were withdrawn from the receptor compartment and analyzed by HPLC. The surface area of the snake skin was 1.8 $cm^2$ and the receptor compartment had a volume of 8-10 ml.

The most remarkable permeation promoting effects compared to Azone® were obtained with clonidine and hydrocortisone where approximately two-fold improvement in permeation was found. Compared to the control, the highest enhancement was obtained with DAIPD for hydrocortisone. The n-octanol/pH 7.0 buffer, partition coefficients of the drugs were reported as log P: 0.4, 0.8, and 1.2 for clonidine, hydrocortisone and indomethacin, respectively. Indomethacin which is the most lipophilic of the three drugs showed an enhancement approximately equal to that of Azone®. It was expected that indomethacin as a more lipophilic compound would exhibit a higher transdermal penetrating effect. However, this order was not followed under the present conditions. These findings suggest that in addition to lipophilicity, other factors such as drug/enhancer and drug/skin interactions also may play a role.

TABLE 2

Effects of DAIPD and Azone® on the penetration of clonidine through shed snake skin at 32°C and pH 7.0 (n=3-5)

| Compound | Slope[a] | Intercept | r[b] | R.E.[c] | Flux[d] | Permeability[e] |
|---|---|---|---|---|---|---|
| DAIPD | 77.92 (4.98)[f] | 2.202 | 0.992 | 27 | 42.86 | $2.14\times10^{e}$ |
| Azone® | 37.56 (0.32) | -28.08 | 0.996 | 13 | 20.84 | $1.04\times10^{-2}$ |
| Control | 2.90 (0.46) | 2.29 | 0.990 | 1 | 1.606 | $0.8\times10^{-4}$ |

[a]Slope of the regression line.
[b]Correlation coefficient of the regression line.
[c]Relative enhancement
[d] ($\mu g/hr\cdot cm^2$)
[e]cm/hr
[f]Standard deviation

TABLE 3

| Effects of DAIPD and Azone® on the penetration of hydrocortisone through shed snake skin at 32°C and pH 7.0 (n=3-5) | | | | | | |
|---|---|---|---|---|---|---|
| Compound | Slope[a] | Intercept | r[b] | R.E.[c] | Flux[d] | Permeability[e] |
| DAIPD | 2.31 (0.35)[f] | -3.24 | 0.989 | 88 | 1.28 | $3.56 \times 10^{-2}$ |
| Azone® | 1.06 (0.31) | -1.37 | 0.998 | 41 | 0.59 | $1.64 \times 10^{-2}$ |
| Control | 0.026 (0.013) | 0.037 | 0.958 | 1 | $1.46 \times 10^{-2}$ | $0.4 \times 10^{-4}$ |

[a]Slope of the regression line.
[b]Correlation coefficient of the regression line.
[c]Relative enhancement
[d] ($\mu g/hr \cdot cm^2$)
[e]cm/hr
[f]Standard deviation

TABLE 4

| Effects of DAIPD and Azone® on the penetration of indomethacin through shed snake skin at 32°C and pH 7.0 (n=3-5) | | | | | | |
|---|---|---|---|---|---|---|
| Compound | Slope[a] | Intercept | r[b] | R.E.[c] | Flux[d] | Permeability[e] |
| DAIPD | 1.89 (0.21)[f] | -0.76 | 0.992 | 53 | 1.05 | $1.14 \times 10^{-2}$ |
| Azone® | 1.66 (0.17) | -1.94 | 0.992 | 46 | 0.92 | $1.01 \times 10^{-2}$ |
| Control | 0.03 (0.0003) | -0.02 | 0.997 | 1 | 0.02 | $0.18 \times 10^{-4}$ |

[a]Slope of the regression line.
[b]Correlation coefficient of the regression line.
[c]Relative enhancement
[d]($\mu g/hr \cdot cm^2$)
[e]cm/hr
[f]Standard deviation

TABLE 5

| Lag times of the permeation of clonidine, hydrocortisone and indomethacin in the presence of DAIPD and Azone® | | |
|---|---|---|
| Drugs | Lag times | (min) |
| | Azone® | DAIPD |
| Clonidine | 45(3)[a] | - |
| Hydrocortisone | 47(5) | 42(5) |
| Indomethacin | 69(7) | 24(7) |

[a]Standard deviation

The penetration profiles of the penetrants examined here showed a lag time followed by a linear steady-state flux. The lag times were calculated according to well-established methods and are listed in TABLE 5.

An examination of lag times alone does not provide an answer for the relatively lower enhancement of indomethacin. No lag time enhancement was found for clonidine in the presence of DAIPD. With hydrocortisone it increases to 45 minutes, whereas with indomethacin it is approximately 24 minutes. Pretreatment with Azone® also shows a similar trend, confirming that besides lipophilicity, other mechanisms influence the fluxes of these drugs.

Examination of the above data as shown in FIG. 1 through FIG. 3 and TABLES 2 through 5 indicates that

compounds of the present invention are topically effective permeation enhancers, that they are useful in enhancing transdermal drug delivery, and that they are more active than conventional prior art enhancers.

EXAMPLE 2

Penetration Promoting Ability of 1-(N,N-dimethylamino)-2-propanol myristate (DAIPM)

The penetration promoting ability of 1-(N,N-dimethylamino)-2-propanol myristate (DAIPM) was examined in a series of experiments similar to those described in Example 1. The results are shown in FIG. 4-FIG. 8 and TABLE 6.

Like DAIPD, DAIPM produced about a two-fold increased in flux over Azone® for clonidine (FIG. 4, cf. TABLE 2 and TABLE 6) and hydrocortisone (FIG. 5, cf. TABLE 3 and TABLE 6). However, DAIPM showed improved performance over DAIPD in producing about a two-fold increased permeability over Azone® for indomethacin (FIG. 6, cf. TABLE 4 and TABLE 6).

TABLE 6

Effects of DAIPM[a] and Azone® on the penetration of some drugs through shed snake skin at 32°C

| Drug | Enhancer | Flux[b] | RE[c] |
|---|---|---|---|
| Prazosin | DAIPM | 0.88 | 19.47 |
| | Azone® | 0.045 | 1 |
| Prostaglandin $E_1$ | DAIPM | 4.84 | 5.76 |
| | Azone® | 0.84 | 1 |
| Clonidine | DAIPM | 1.21 | 1.90 |
| | Azone® | 0.64 | 1 |
| Indomethacin | DAIPM | 31.14 | 2.19 |
| | Azone® | 14.21 | 1 |
| Hydrocortisone | DAIPM | 4.92 | 2.0 |
| | Azone® | 2.47 | 1 |

[a] 1-(N,N-dimethylamino)-2-propanol myristate
[b] (μg/hr·cm$^2$)
[c] Relative enhancement compared to Azone®

DAIPM produced even more substantial improvements over Azone® in experiments with two other drugs. DAIPM enhanced the penetration of prostaglandin $E_1$ (FIG. 7) nearly four-fold compared to Azone® (TABLE 6). A nearly twenty-fold enhancement was found in experiments with the drug Prazosin (FIG. 8, TABLE 6).

EXAMPLE 3

Biodegradability of 1-(N,N-dimethylamino)-2-propanol dodecanoate (DAIPD)

A 0.1 ml aliquot of porcine esterase (235 units per mg protein) was diluted to 100 ml with pH 7.0 phosphate buffer. The enhancer solution was prepared by dissolving -12 mg (-0.045 mmoles) of the enhancer in 10 ml acetonitrile. An aliquot of 100 μl of this solution was transferred into a 10 ml volumetric flask. 9.8 ml of pH 7.0 buffer and 100 μl diluted esterase solution were added. The mixture was kept in a water bath at 32°C with constant stirring. The disappearance of the enhancer peak was monitored by HPLC. The absorbance wavelength was 204 nm. The solvent system was a mixture of acetonitrile and 0.02 M aqueous sodium hexanesulfonate (7:4) with a flow rate of 0.9 ml/min. The retention time of DAIPD was 3.95 min. The kinetic runs were done in triplicate. The results are shown in FIG. 9.

The existence of esterase activity has been reported in shed snake skin (Nghiem, B.T., and Higuchi, T., Esterase activity in snake skin, Int. J. Pharm. 44: 125-130, 1988) . To confirm the biodegradability of DAIPD, its fragmentation in the presence of porcine esterase was examined. The logarithm of the peak heights versus time indicates that the degradation follows pseudo first order kinetics, with a $k_{obs}$ of 0.0087 min$^{-1}$ and $t_{1/2}$ of 79.5 min (FIG. 9). Esterase-catalyzed biodegradability of the enhancer is therefore confirmed. Hirvonen et al., below, showed that the transdermal delivery of propranolol is no longer enhanced following pretreatment with dodecyl N,N-dimethylamino acetate (DDAA) , a biodegradable enhancer described in U.S. Patents No. 4,980,378 and No. 5, 082, 866, after 4 days whereas with Azone® the enhancement still existed after a week. (Hirvonen, J., Paronen, P., and Urtti, A., Reversible enhancement

of transdermal propranolol by dodecyl N,N-dimethylamino acetate, 18th International Symposium on Controlled Release of Bioactive Materials, July 8-11, 1991, Amsterdam, p 31). Therefore, if they exist, the irritating and toxic effects of biodegradable can considerably reduced and the activity period will be limited.

Overall, the results show that DAIPD is a biodegradable enhancer which is more effective than Azone®. DAIPD potentially may be useful in the permeation enhancement of several different classes of drugs.

EXAMPLE 4

Synthesis of 1-(N,N-dimethylamino)-2-propanol oleate (DAIPO)

1-(N,N-dimethylamino)-2-propanololeate (DAIPO) is an example of the compounds of formula (II).

To 10.3 g (0.1 M) of 1-(N,N-dimethylamino)-2-propanol in 250 ml $CHCl_3$ in the presence of triethylamine (15 ml), 50 g (0.1 M) of oleoyl chloride (60%) was added incrementally and stirred for 24 hr at room temperature. After filtration of the residue, the reaction mixture was washed three times with water (250 ml each washing) and the organic phase was dried over anhydrous magnesium sulfate. The solvent was evaporated in vacuo, the oily residue was dissolved in ethyl acetate, and purified by column chromatography using silica gel as supporting material and the same solvent as eluent. The procedure was carried out in darkness and the compound was kept under nitrogen. The reaction course was checked by thin layer chromatography (TLC). Ethyl acetate was used as the solvent for TLC analysis. The visualization was accomplished using iodine vapors. The yield was 60%. The TLC Rf value was 0.24 (ethyl acetate). IR ($CHCl_3$) : $\gamma$ 2925,2845 (C-H) , 1730 (C=O) , 1090 (C-O-C) $cm^{-1}$; 1H-NMR ($CDCl_3$) $\delta$ 0.88 (3H, t, $CH_3$) , 1.21-1.23 (3H, d, $CH_3$-CH), 1.32 (broad s, 22H, $(CH_2)_{11}$), 1.98-2.08 (4H, m, 2 ($CH_2$-CH=)), 2.25 (6H, s, $N(CH_3)_2$) , 2.29 (2H, t, $CH_2$-N), 2.46-2.52 (2H, m, $CH_2$-CO), 5.02-5.08 (1H, m, CH-$CH_3$), 5.32-5.38 (2H, d, CH=CH) ppm; MS(EI):m/z (RA %) 367(38), 158 (15) , 145(20), 102(10), 58 (100) ; $C_{23}H_{45}NO_2$ (367.61) requires 367.

FIG. 10 shows enhancement data by means of penetration profiles of clonidine with an unsaturated ester of formula (II), DAIPO, in comparison with oleic acid, with DDAA, and with a control, using the skin penetration procedures and model described in Example 1. The data reveal that these compounds are generally more potent in enhancing the absorption of basic molecules than those of the series disclosed by U.S. Patents No. 4,980,378 and No. 5,082,866, and that they are effective in enhancing the penetration of neutral and acidic compounds.

It can therefore be seen that the invention accomplishes all of its stated objectives.

The foregoing discussion and the accompanying examples are presented as illustrative, and are not to be taken as limiting. Still other variations within the spirit and scope of this invention are possible and will readily present themselves to those skilled in the art.

## Claims

**1.** A pharmacological composition for topical administration of a pharmacologically active topical medicament, said composition comprising:

(a) a pharmacologically active topical medicament in an amount sufficient to achieve the desired physiological effect; and
(b) an active agent skin penetration enhancing compound of the formula:

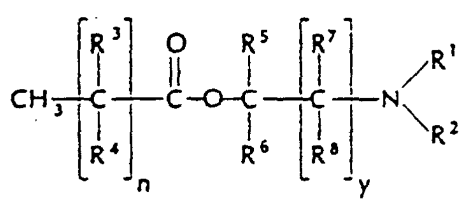

(I)

wherein n is an integer having a value in the range of 5 to 18; y is an integer having a value in the range of 0 to 5; and $R^1$, $R^2$, $R^3$, $R^4$, $R^5$, $R^6$, and $R^7$ can be alike or different from one another and are members of the group consisting of hydrogen, and $C_1$- to $C_8$- alkyl; and $R^8$ is a member of the group consisting of hydrogen, hydroxyl and $C_1$- to $C_8$- alkyl.

**2.** The composition of claim 1 wherein $R^1$ and $R^2$ are members of the group consisting of hydrogen, methyl and ethyl.

**3.** The composition of claim 1 wherein $R^1$ and $R^2$ are methyl.

**4.** The composition of claim 1 wherein the penetration enhancer of formula (I) is 1-(N,N-dimethylamino)-2-propanol dodecanoate.

**5.** The composition of claim 1 wherein the penetration enhancer of formula (I) is 1-(N,N-dimethylamino)-2-propanol myristate.

**6.** A pharmacological composition for topical administration of a pharmacologically active topical medicament, said composition comprising:

(a) a pharmacologically active topical medicament in an amount sufficient to achieve the desired physiological effect; and
(b) an active agent skin penetration enhancing compound of the formula:

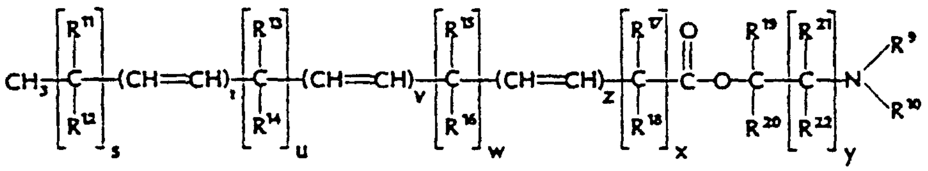

(II)

wherein t, v, and z each is an integer having a value in the range of 0 to 1 inclusive, such that (t + v + z) equals an integer of at least 1, s, u, w, and x each is an integer having a value in the range of 0 to 12 inclusive, such that (s + u + w + x) equals an integer in the range of 4 to 18 inclusive, y is an integer having a value in the range of 0 to 5 inclusive, and $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, and $R^{22}$ can be alike or different from one another and are members of the group consisting of hydrogen, and $C_1$- to $C_8$- alkyl.

**7.** The composition of claim 6 wherein $R^9$ and $R^{11}$ are members of the group consisting of hydrogen, methyl and ethyl.

**8.** The composition of claim 6 wherein $R^9$ and $R^{10}$ are methyl.

**9.** The composition of claim 6 wherein the compound is 1-(N,N-dimethylamino)-2-propanol oleate.

**10.** A composition according to any one of claims 1 to 9, said composition being formulated as a cream, lotion, gel, ointment, suppository, spray, aerosol, buccal tablet, sublingual tablet or as a buccal, gingival, sublingual or palatal patch.

**Patentansprüche**

**1.** Pharmakologische Zusammensetzung zur topischen Verabreichung eines pharmakologisch wirksamen topischen Arzneimittels, wobei die Zusammensetzung umfasst:

(a) ein pharmakologisch wirksames topisches Arzneimittel in einer Menge, die ausreicht, um die gewünschte physiologische Wirkung zu erreichen, und

(b) eine die Hautpenetration eines wirksamen Mittels verstärkende Verbindung der Formel:

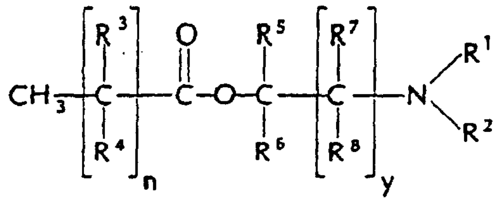

(I)

worin n eine ganze Zahl mit einem Wert von 5 bis 18 ist, y eine ganze Zahl mit einem Wert von 0 bis 5 ist und $R^1$, $R^2$, $R^3$, $R^4$, $R^5$, $R^6$ und $R^7$ gleich oder voneinander verschieden sein können und Mitglieder sind aus der Gruppe bestehend aus Wasserstoffatom und $C_1$- bis $C_8$-Alkylgruppen und $R^8$ ein Mitglied ist aus der Gruppe bestehend aus Wasserstoffatom, Hydroxyl- und $C_1$- bis $C_8$-Alkylgruppen.

**2.** Zusammensetzung nach Anspruch 1, wobei $R^1$ und $R^2$ Mitglieder sind aus der Gruppe bestehend aus Wasserstoffatom, Methyl- und Ethylgruppen.

**3.** Zusammensetzung nach Anspruch 1, wobei $R^1$ und $R^2$ Methylgruppen sind.

**4.** Zusammensetzung nach Anspruch 1, wobei das Penetrationsverstärkungsmittel der Formel (I) 1-(N,N-Dimethylamino)-2-propanoldodecanoat ist.

**5.** Zusammensetzung nach Anspruch 1, wobei das Penetrationsverstärkungsmittel der Formel (I) 1-(N,N-Dimethylamino)-2-propanolmyristat ist.

**6.** Pharmakologische Zusammensetzung zur topischen Verabreichung eines pharmakologisch wirksamen topischen Arzneimittels, wobei die Zusammensetzung umfasst:

(a) ein pharmakologisch wirksames topisches Arzneimittel in einer Menge, die ausreicht, um die gewünschte physiologische Wirkung zu erreichen, und

(b) eine die Hautpenetration eines wirksamen Mittels verstärkende Verbindung der Formel:

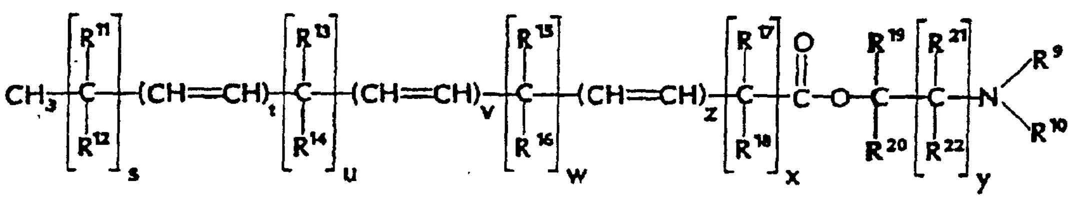

(II)

worin t, v und z jeweils eine ganze Zahl mit einem Wert von O bis einschließlich 1 derart sind, dass (t + v + z) eine ganze Zahl von mindestens 1 ergibt, s, u, w und x jeweils eine ganze Zahl mit einem Wert von 0 bis einschließlich 12 derart sind, dass (s + u + w + x) eine ganze Zahl von 4 bis einschließlich 18 ergibt, y eine ganze Zahl mit einem Wert von 0 bis einschließlich 5 ist und $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$ und $R^{22}$ gleich oder voneinander verschieden sein können und Mitglieder sind aus der Gruppe bestehend aus Wasserstoffatom und $C_1$- bis $C_8$-Alkylgruppen.

**7.** Zusammensetzung nach Anspruch 6, wobei $R^9$ und $R^{10}$ Mitglieder sind aus der Gruppe bestehend aus Wasserstoffatom, Methyl- und Ethylgruppen.

**8.** Zusammensetzung nach Anspruch 6, wobei $R^9$ und $R^{10}$ Methylgruppen sind.

**9.** Zusammensetzung nach Anspruch 6, wobei die Verbindung 1-(N,N-Dimethylamino)-2-propanololeat ist.

**10.** Zusammensetzung nach einem der Ansprüche 1 bis 9, wobei die Zusammensetzung als eine Creme, Lotion, ein Gel, eine Salbe, ein Suppositorium, Spray, Aerosol, eine buccale Tablette, sublinguale Tablette oder als ein buccales Pflaster, Zahnfleischpflaster, sublinguales Pflaster oder Gaumenpflaster formuliert ist.

## Revendications

1. Composition pharmacologique pour administration topique d'un médicament topique à activité pharmacologique, ladite composition comprenant:

   (a) un médicament topique à activité pharmacologique en une quantité suffisante pour atteindre l'effet physiologique désiré; et
   (b) un composé pour le renforcement de la pénétration de l'agent actif dans la peau, de formule:

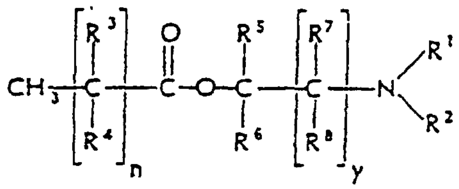

(I)

où n est un entier ayant une valeur dans l'intervalle de 5 à 18; y est un entier ayant une valeur dans l'intervalle de 0 à 5; et $R^1$, $R^2$, $R^3$, $R^4$, $R^5$, $R^6$ et $R^7$ peuvent être identiques ou différents l'un de l'autre et sont des membres du groupe consistant en l'hydrogène et un alkyle en $C_1$ à $C_8$; et $R^8$ est un membre du groupe consistant en l'hydrogène, l'hydroxyle et un alkyle en $C_1$ à $C_8$.

2. Composition selon la revendication 1, dans laquelle $R^1$ et $R^2$ sont des membres du groupe consistant en l'hydrogène, le méthyle et l'éthyle.

3. Composition selon la revendication 1, dans laquelle $R^1$ et $R^2$ sont le méthyle.

4. Composition selon la revendication 1, dans laquelle le renforçateur de pénétration de formule (I) est le dodécanoate de 1-(N,N-diméthylamino)-2-propanol.

5. Composition selon la revendication 1, dans laquelle le renforçateur de pénétration de formule (I) est le myristate de 1-(N,N-diméthylamino)-2-propanol.

6. Composition pharmacologique pour administration topique d'un médicament topique à activité pharmacologique, ladite composition comprenant:

   (a) un médicament topique à activité pharmacologique en une quantité suffisante pour atteindre l'effet physiologique désiré; et
   (b) un composé pour le renforcement de la pénétration de l'agent actif dans la peau, de formule:

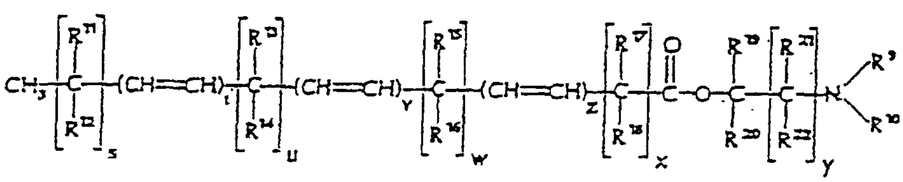

(II)

où t, v et z sont chacun un entier ayant une valeur dans l'intervalle de 0 à 1 inclusivement, de telle sorte que (t + v + z) soit égal à un entier d'au moins 1, s,u, w et x sont chacun un entier ayant une valeur dans l'intervalle de 0 à 12 inclusivement, de telle sorte que (s + u + w + x) soit égal à un entier dans l'intervalle de 4 à 18 inclusivement, y est un entier ayant une valeur dans l'intervalle de 0 à 5 inclusivement, et $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$ et $R^{22}$ peuvent être identiques ou différents l'un de l'autre et sont des membres du groupe consistant en l'hydrogène, et un alkyle en $C_1$ à $C_8$.

7. Composition selon la revendication 6, dans laquelle $R^9$ et $R^{10}$ sont des membres du groupe consistant en l'hydrogène, le méthyle et l'éthyle.

8. Composition selon la revendication 6, dans laquelle $R^9$ et $R^{10}$ sont le méthyle.

9. Composition selon la revendication 6, dans laquelle le composé est l'oléate de 1-(N,N-diméthylamino)-2-propanol.

10. Composition selon l'une quelconque des revendications 1 à 9, ladite composition étant formulée en crème, lotion, gel, pommade, suppositoire, spray, aérosol, comprimé gingivo-jugal, comprimé sublingual, ou en timbre buccal, gingival, sublingual ou palatal.

# Fig. 1

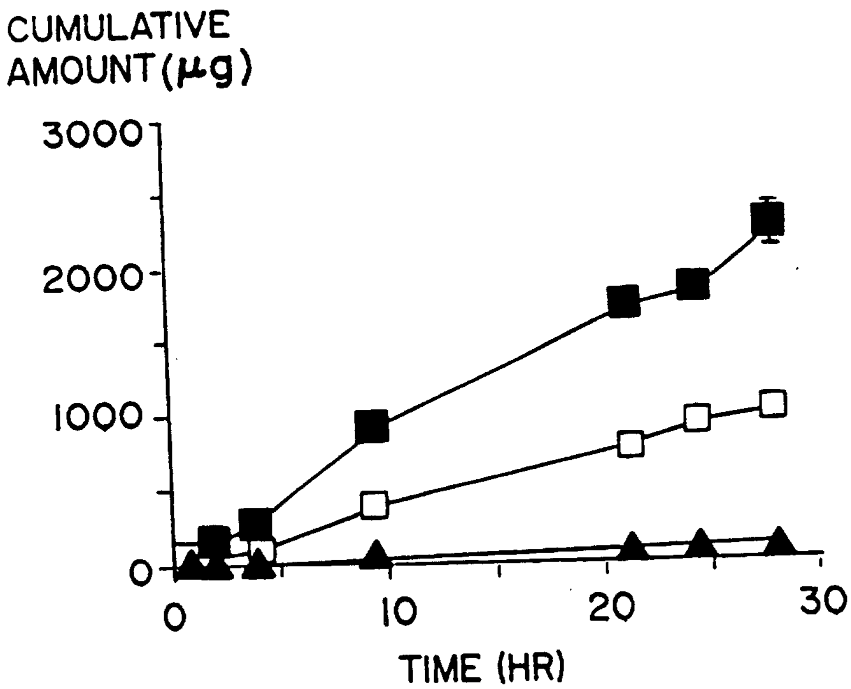

# Fig. 2

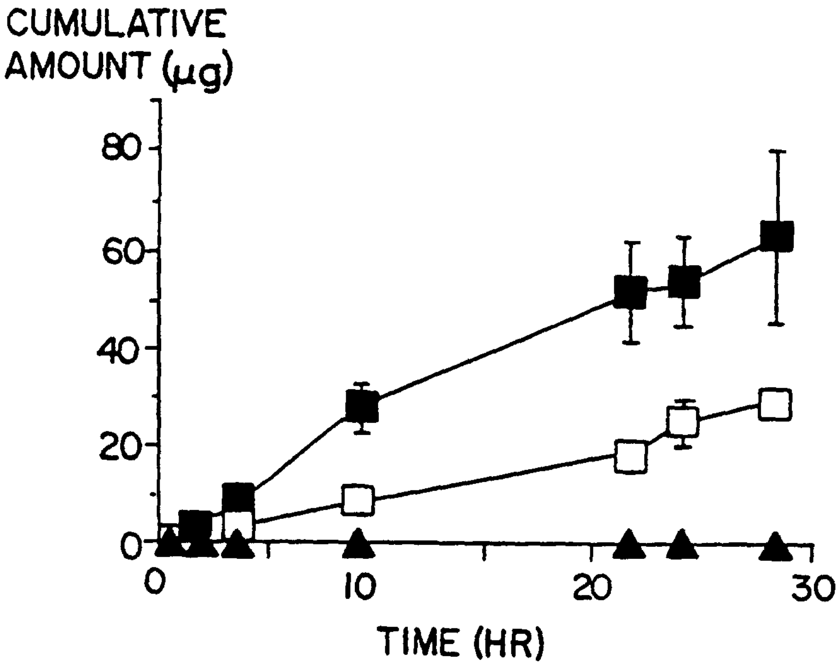

Fig. 3

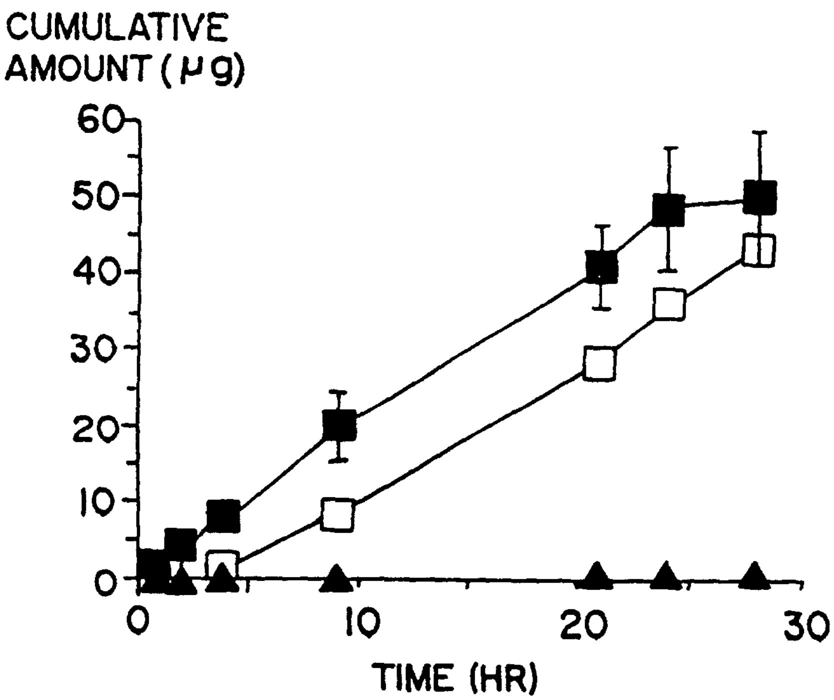

# Fig. 4

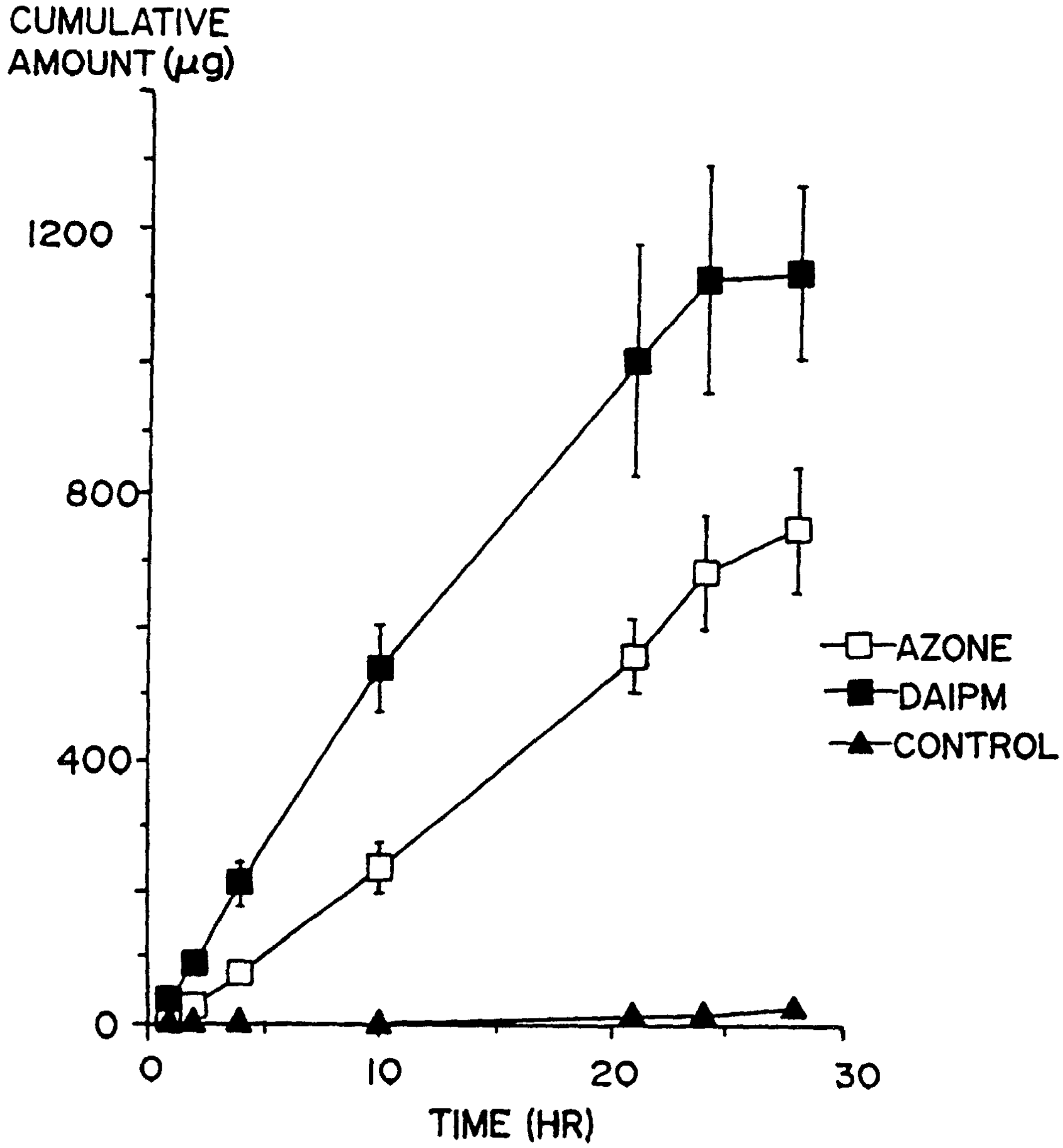
CUMULATIVE AMOUNT (μg)
1200
800
400
0
0
10
20
30
TIME (HR)
AZONE
DAIPM
CONTROL

Fig. 5

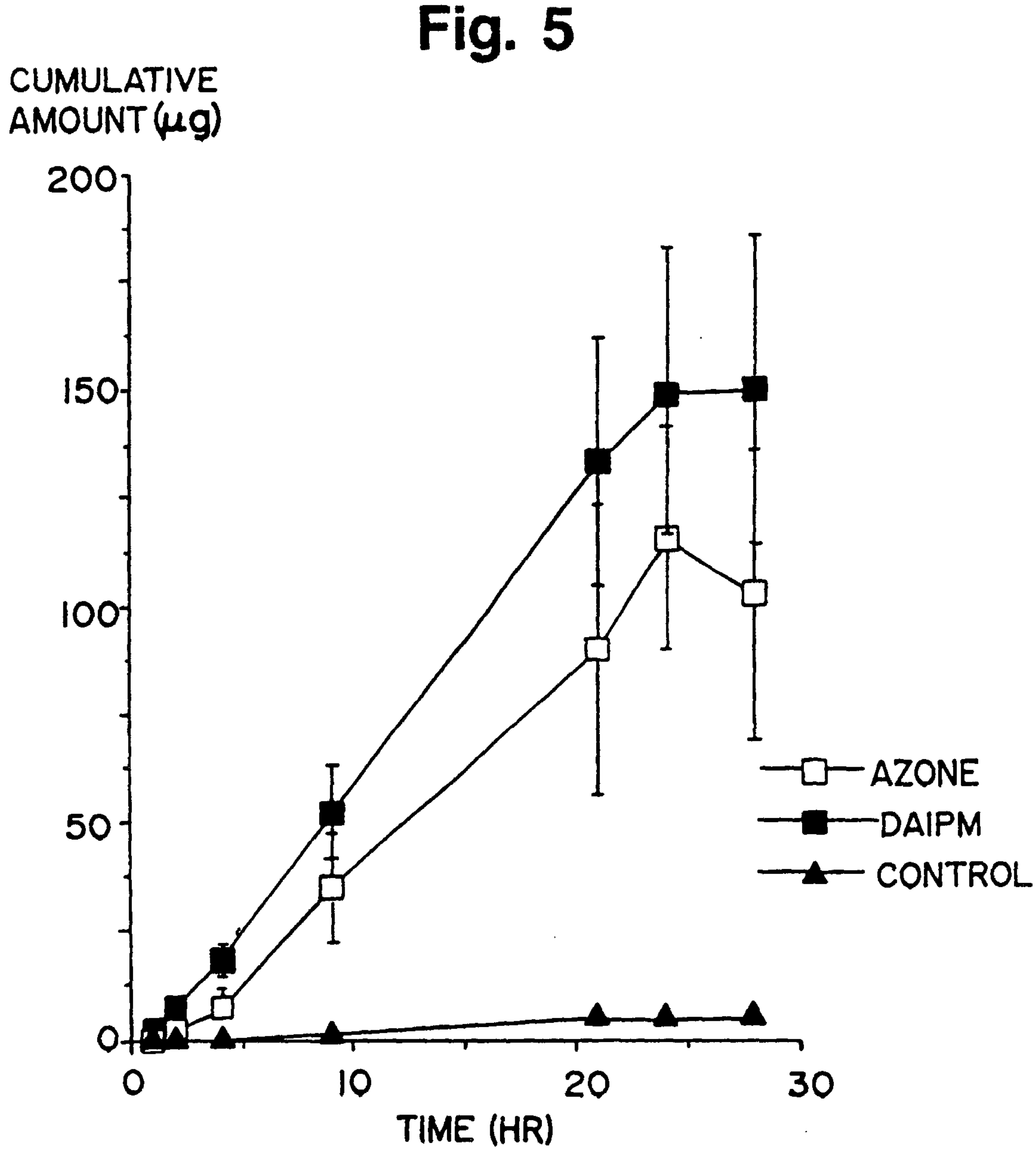
CUMULATIVE AMOUNT (µg)
200
150
100
50
0
0
10
20
30
TIME (HR)
AZONE
DAIPM
CONTROL

# Fig. 6

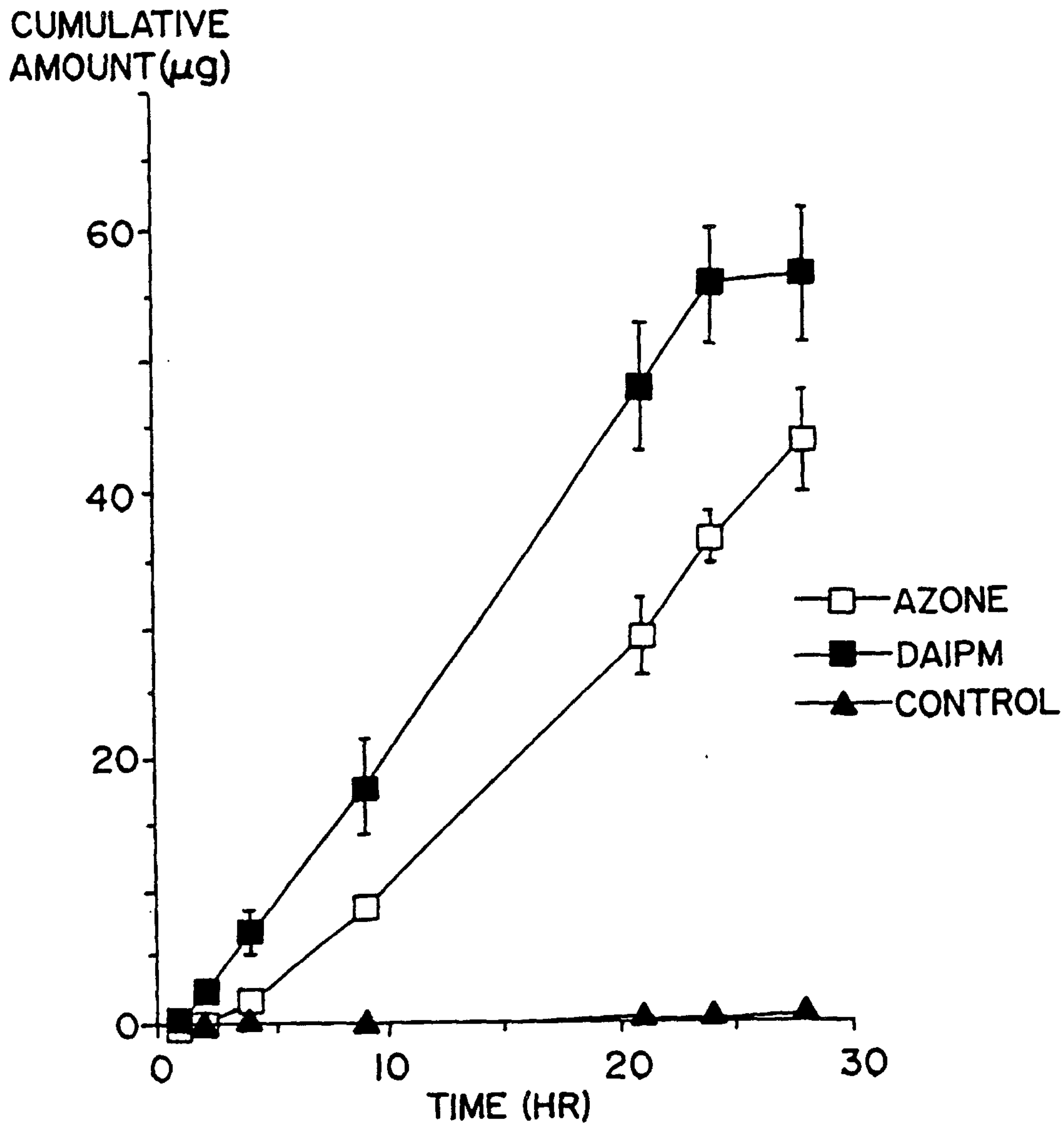
CUMULATIVE
AMOUNT(μg)
60
40
20
0
0
10
20
30
TIME (HR)
AZONE
DAIPM
CONTROL

# Fig. 7

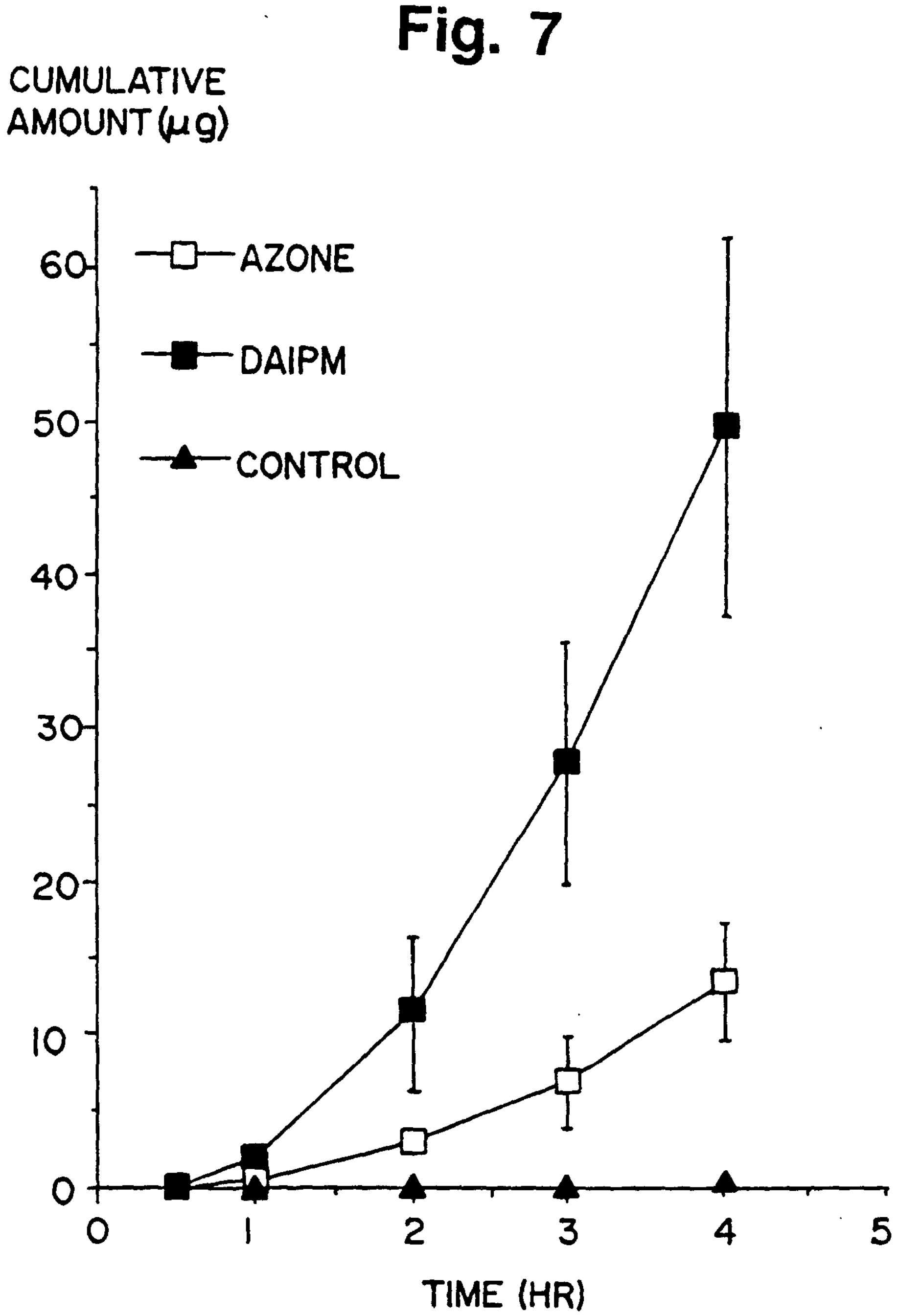
CUMULATIVE AMOUNT (μg)
60
50
40
30
20
10
0
AZONE
DAIPM
CONTROL
0
1
2
3
4
5
TIME (HR)

Fig. 8

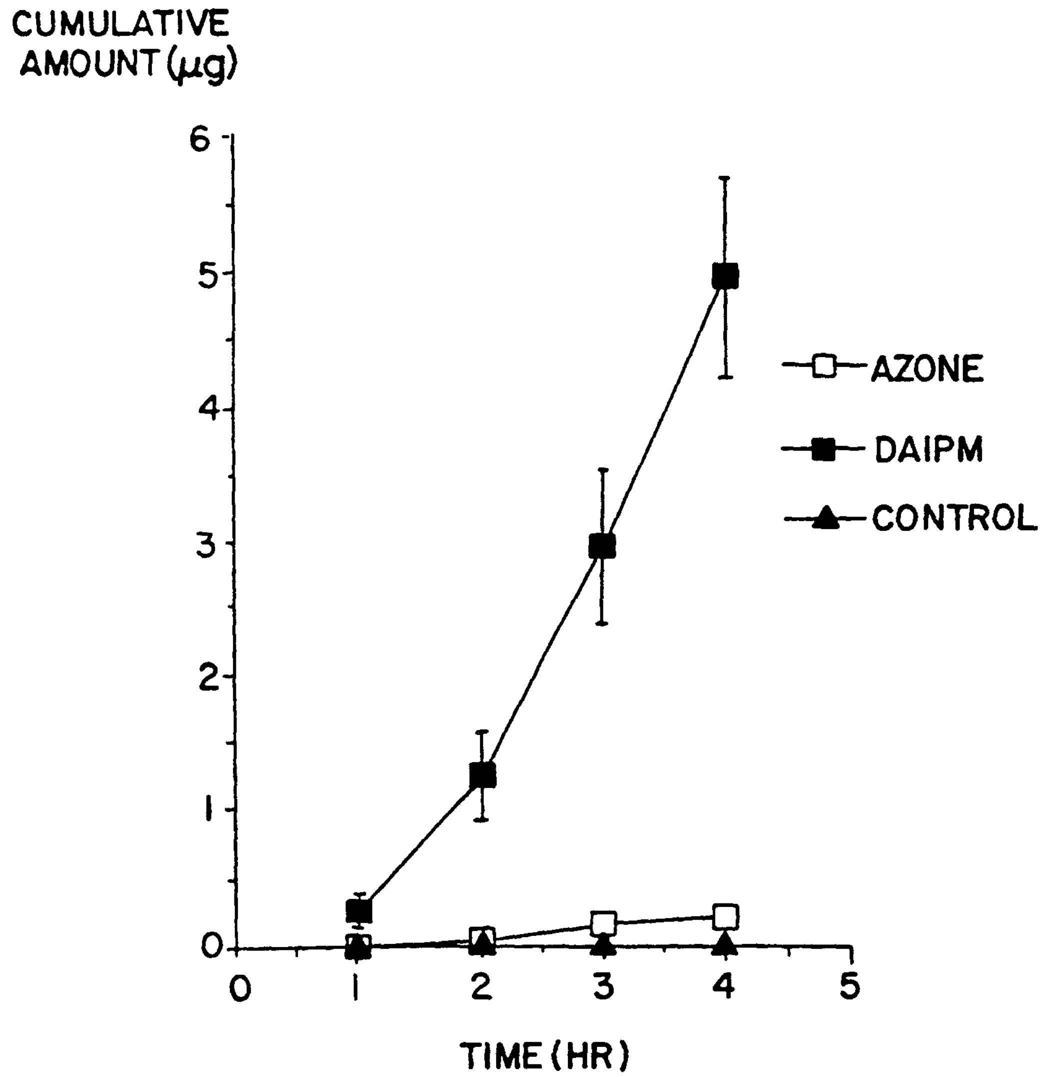
CUMULATIVE AMOUNT (μg)
6
5
4
3
2
1
0
0
1
2
3
4
5
TIME (HR)
AZONE
DAIPM
CONTROL

Fig. 9

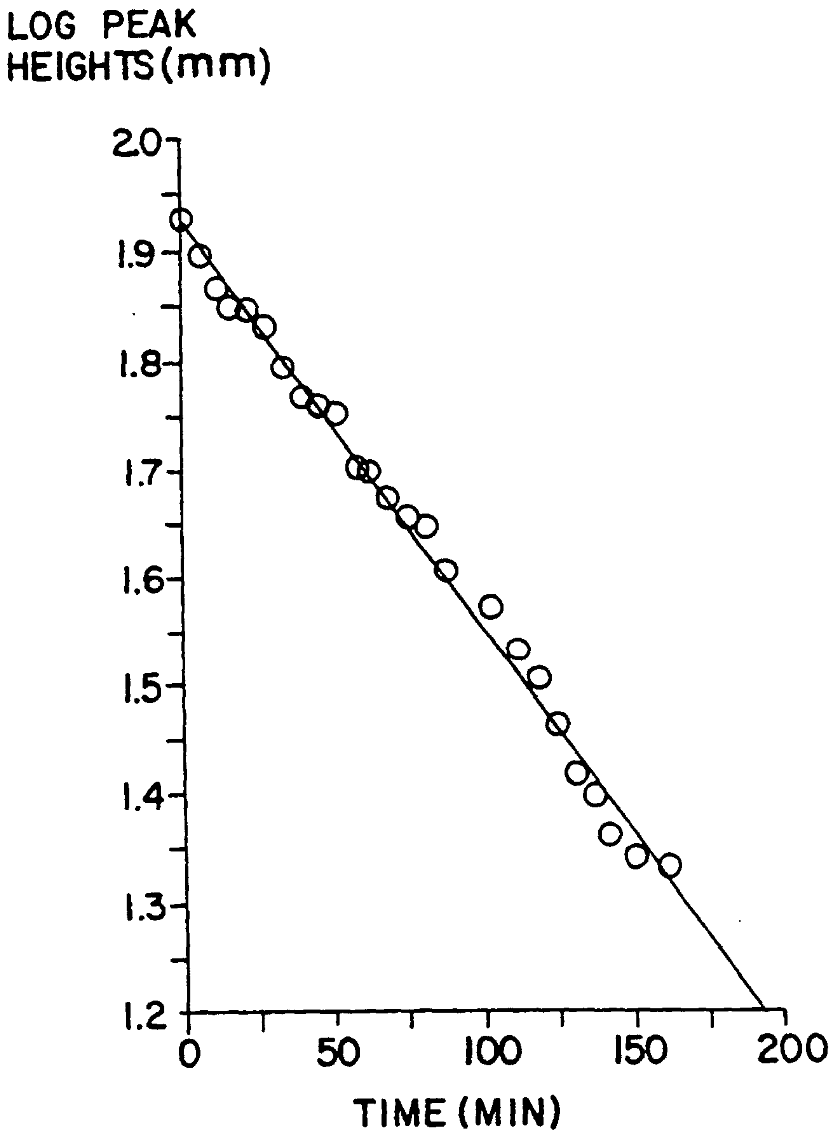
LOG PEAK
HEIGHTS (mm)
2.0
1.9
1.8
1.7
1.6
1.5
1.4
1.3
1.2
0
50
100
150
200
TIME (MIN)

# Fig. 10

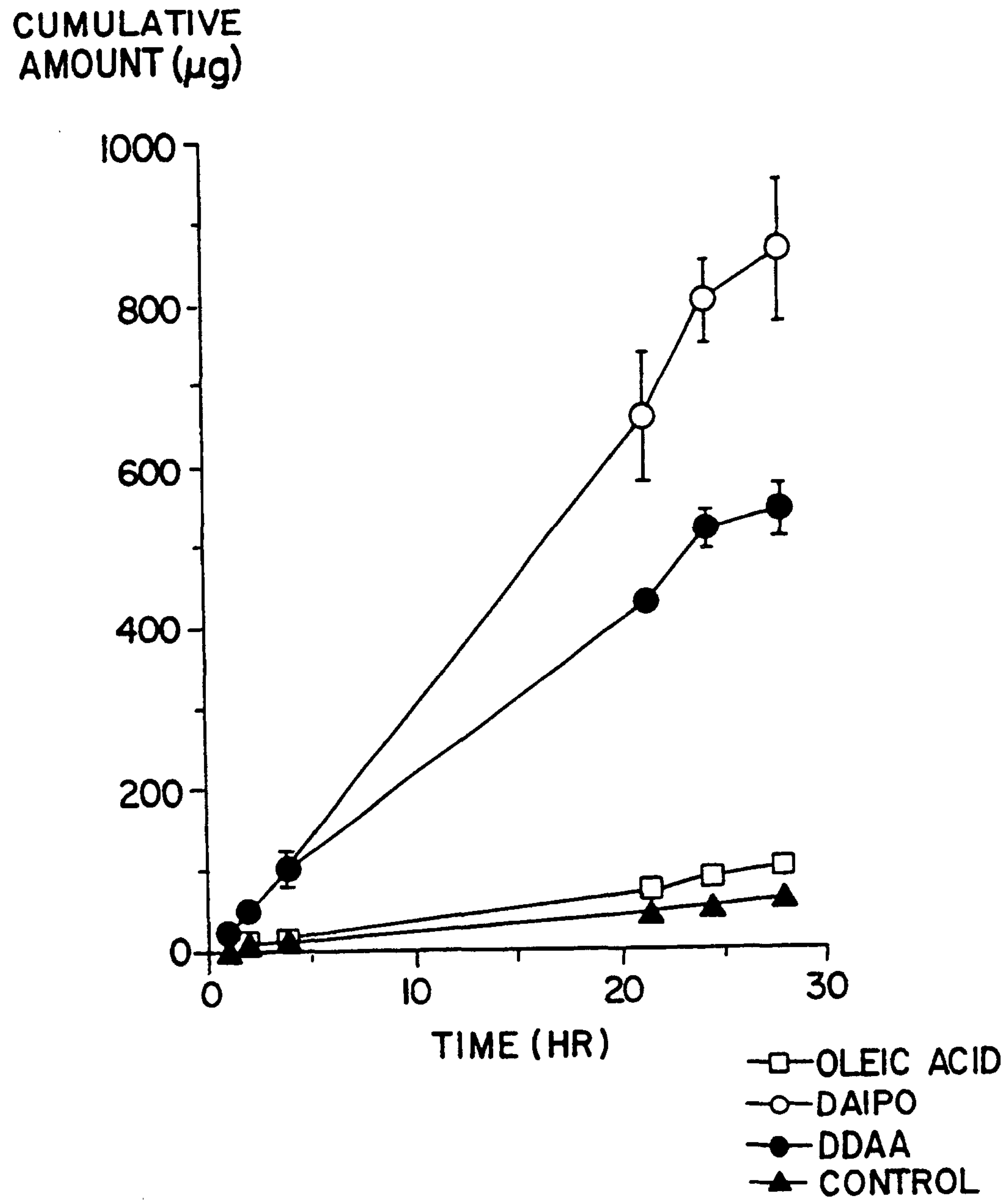
CUMULATIVE AMOUNT (μg)
1000
800
600
400
200
0
0
10
20
30
TIME (HR)
OLEIC ACID
DAIPO
DDAA
CONTROL